# EUROPEAN PATENT SPECIFICATION

(11) **EP 2 121 053 B1**
(45) Date of publication and mention of the grant of the patent: **24.07.2013**
(21) Application number: 07866578.3
(22) Date of filing: 21.12.2007
(51) Int. Cl.: A61L 27/02, A61L 27/56

(54) **METAL OXIDE SCAFFOLDS**
METALLOXIDGERÜST
ECHAFAUDAGE D'OXYDE MÉTALLIQUE

(30) Priority: 21.12.2006 US 876150 P
(43) Date of publication of application: 25.11.2009
(73) Proprietor: Corticalis AS, 1450 Nesoddtangen (NO)
(72) Inventor: LYNGSTADAAS, Stale, Petter, N-1450 Nesoddtangen (NO); HAUGEN, Havard, Jostein, 0376 Oslo (NO); ELLINGSEN, Jan, Eirik, N-1356 Bekkestua (NO)
(74) Representative: Valea AB
(86) International application number: PCT/IB2007/004062
(87) International publication number: WO 2008/078164

(56) References cited:
- EP-A- 0 116 298
- EP-A- 1 270 533
- DE-A1- 10 244 439
- US-A1- 2005 161 440
- H. TIAINEN ET AL.: "Ultra-porous titanium oxide scaffold with high compressive strength", J MATER SCI: MATER MED, vol. 21, 14 August 2010 (2010-08-14), pages 2783-2792, DOI: 10.2007/s10856-010-4142-1

## Description

### FIELD OF THE INVENTION

The present invention relates to the field of implants comprising materials with improved properties in terms of mechanical stability and biocompatibility for implantation into a subject. In particular the invention relates to implants comprising porous ceramic and metal materials and a method of making such materials for use as an osteoconductive and osseointegrating material.

### BACKGROUND OF THE INVENTION

Conditions such as trauma, tumours, cancer, periodontitis and osteoporosis may lead to bone loss, reduced bone growth and volume. For these and other reasons it is of great importance to find methods to improve bone growth and to regain bone anatomy. Scaffolds may be used as a framework for the cells participating in the bone regeneration process, but also as a framework as a substitute for the lost bone structure. It is also of interest to provide a scaffold to be implanted into a subject with a surface structure that stimulates the bone cells to grow to allow a coating of the implanted structure by bone after a healing process

Orthopedic implants are utilized for the preservation and restoration of the function in the musculoskeletal system, particularly joints and bones, including alleviation of pain in these structures. Vascular stents are tubular implants arranged for insertion into blood vessels in order to prevent or counteract a localized flow constriction, i.e. they counteract significant decreases in blood vessel diameter.

Orthopedic implants are commonly constructed from materials that are stable in biological environments and that withstand physical stress with minimal deformation. These materials must possess strength, resistance to corrosion, have a good biocompatibility and have good wear properties. Materials which fulfill these requirements include biocompatible materials such as titanium and cobolt-chrome alloy.

For the purposes of tissue engineering it is previously known to use scaffolds to support growth of cells. It is believed that the scaffold pore size, porosity and interconnectivity are important factors that influence the behaviour of the cells and the quality of the tissue regenerated. Prior art scaffolds are typically made of calcium phosphates, hydroxyl apatites and of different kinds of polymers.

One principle of tissue engineering is to harvest cells, expand the cell population *in vitro,* if necessary, and seed them onto a supporting three-dimensional scaffold, where the cells can grow into a complete tissue or organ [1-5]. For most clinical applications, the choice of scaffold material and structure is crucial [6-8]. In order to achieve a high cell density within the scaffold, the material needs to have a high surface area to volume ratio. The pores must be open and large enough such that the cells can migrate into the scaffolds. When cells have attached to the material surface there must be enough space and channels to allow for nutrient delivery, waste removal, exclusion of material or cells and protein transport, which is only obtainable with an interconnected network of pores [9, 10]. Biological responses to implanted scaffolds are also influenced by scaffold design factors such as three-dimensional microarchitecture [11]. In addition to the structural properties of the material, physical properties of the material surface for cell attachment are essential.

Titanium and titanium alloys are frequently used as implant materials in dental and orthopedic surgery due to their biocompatibility with bone tissue and their tendency to form a firm attachment directly with bone tissue. This interaction between bone tissue and metal leading to this firm attachment is called "osseointegration".

Some of the metals or alloys, such as titanium, zirconium, hafnium, tantalum, niobium, or alloys thereof, that are used for bone implants are capable of forming a relatively strong bond with the bone tissue, a bond which may be as strong as the bone tissue per se, or sometimes even stronger.

Although the bond between the metal, e.g. titanium and the bone tissue may be comparatively strong, it is often desirable to enhance this bond.

To date there are several methods for treating metallic implants in order to obtain a better attachment of the implant, and thus improved osseointegration. Some of these involve altering the morphology of the implant, for example by creating relatively large irregularities on the implant surface in order to increase the surface roughness in comparison to an untreated surface. An increased surface roughness gives a larger contact and attachment area between the implant and the bone tissue, whereby a better mechanical retention and strength may be obtained. A surface roughness may be provided by, for example, plasma spraying, blasting or etching.

Rough etching of implant surfaces may be performed with reducing acids, such as hydrofluoric acid (HF) or mixtures of hydrochloric acid (HCl) and sulfuric acid (H₂SO₄). The aim of such a rough etching process is to obtain implant surfaces with rather large irregularities, such as pore diameters within the range of 2-10 µm and pore depths within the range of 1-5 µm.

Other methods involve altering of the chemical properties of the implant surface. This may e.g. be done by using low concentrated fluoride solutions, e.g. HF or NaF, to modify the surface chemistry as well as in same occasions the surface nano structure. For example one such method involves the application of a layer of ceramic material such as hydroxyapatite to the implant surface, inter alia, in order to stimulate the regeneration of the bone tissue. Ceramic coatings however may be brittle and may flake or break off from the implant surface, which may in turn lead to the ultimate failure of the implant.

Besides the above disclosed methods of implant surface modification, it shall be noted that in contact with oxygen, titanium, zirconium, hafnium, tantalum, niobium and their alloys are instantaneously covered with a thin oxide layer. The oxide layers of titanium implants mainly consist of titanium(IV)dioxide (TiO₂) with minor amounts of Ti₂O₃ and TiO. The titanium oxide generally has a thickness of about 4-8 nm.

WO 95/17217 and WO 94/13334 describe different processes for treating a metallic implant with an aqueous solution comprising fluoride. Both these prior applications describe metallic implants having improved biocompatibility, and methods for producing such metallic implants. Specifically, the rate of bone tissue attachment is increased and a stronger bonding between the implant and the bone tissue is obtained. The improved biocompatibility of these implants is believed to be due to retaining of fluorine and/or fluoride on the implant surfaces.

Fluorine and/or fluoride is, according to J E Ellingsen, "Pre-treatment of titanium implants with fluoride improves their retention in bone", Journal of Material Science: Materials in Medicine, 6 (1995), pp 749-753, assumed to react with the surface titanium oxide layer and replace titanium bound oxygen to form a titanium fluoride compound. *In vivo,* the oxygen of phosphate in tissue fluid may replace the fluoride in the oxide layer and the phosphate will then become covalently bound to the titanium surface. This may induce a bone formation where phosphate in the bone is bound to the titanium implant. Moreover, the released fluoride may catalyse this reaction and induce formation of fluoridated hydroxyapatite and fluorapatite in the surrounding bone.

WO 04/008983 and WO 04/008984 disclose further methods for improving the biocompatibility of an implant. WO 04/008983 discloses a method for treating implants comprising providing fluorine and/or fluoride on the implant surface and providing a micro-roughness on the surface having a root-mean-square roughness (Rq and/or Sq) of ≤ 250nm and/or providing pores having a pore diameter of ≤ 1 µm and a pore depth of ≤ 500 nm. WO 04/008984 discloses a method for treating a metallic implant surface to provide a micro-roughness with pores having a pore diameter of ≤ 1 µm and a pore depth of ≤ 500 nm and a peak width, at half the pore depth of from 15 to 150% of the pore diameter.

Bone in-growth is known to preferentially occur in highly porous, open cell structures in which the cell size is roughly the same as that of trabecular bone (approximately 0.25-0.5 mm), with struts roughly 100 µm (0.1 mm) in diameter. Materials with high porosity and possessing a controlled microstructure are thus of interest to both orthopaedic and dental implant manufacturers. For the orthopedic market, bone in-growth and on-growth options currently include the following: (a) DePuy Inc. sinters metal beads to implant surfaces, leading to a microstructure that is controlled and of a suitable pore size for bone in-growth, but with a lower than optimum porosity for bone in-growth; (b) Zimmer Inc. uses fiber metal pads produced by diffusion bonding loose fibers, wherein the pads are then diffusion bonded to implants or insert injection molded in composite structures, which also have lower than optimum density for bone in-growth; (c) Biomet Inc. uses a plasma sprayed surface that results in a roughened surface that produces on-growth, but does not produce bone in-growth; and (d) Implex Corporation produces using a chemical vapor deposition process to produce a tantalum-coated carbon microstructure that has also been called a metal foam. Research has suggested that this "trabecular metal" leads to high quality bone in-growth. Trabecular metal has the advantages of high porosity, an open-cell structure and a cell size that is conducive to bone in-growth. However, trabecular metal has a chemistry and coating thickness that are difficult to control. Trabecular metal is very expensive, due to material and process costs and long processing times, primarily associated with chemical vapor deposition (CVD). Furthermore, CVD requires the use of very toxic chemicals, which is disfavored in manufacturing and for biomedical applications.

Scaffolds available today are often resorbable, which means that they are degraded after implantation into a subject. Although this may be preferable in some cases, in other cases it may be a disadvantage as it also results in the loss of a stabilizing function of the implant itself. Also prior art scaffolds often trigger inflammatory responses and causes infections. For example, bone implant scaffolds of animal origin, may cause allergic reactions when implanted into another animal. Metal implants with a passivating oxide layer have a good biocompatibility which means that the above mentioned disadvantages may be overcome by the use of implants made of such materials. However, it has previously not been possible to produce metal oxide scaffolds comprising titanium oxide that have a mechanical stability high enough to be practically useful.

One of the most promising biocompatible materials in this sense has been proven in previous studies to be a bioactive ceramic, TiO₂ [25 -28]. This material has shown particular biocompatible properties, where scaffolds were implanted in rats for 55 weeks without any signs of inflammatory responses or encapsuling [28]. Little work has been made in the three-dimensional open pore manufacturing of titanium dioxide [29, 30]. The objective of this work was to produce ceramic foams with their defined macro-, micro- and nano-structures and to show the possible application of ceramic foams as scaffolds for cell cultures.

DE 102 44 439 A discloses a ceramic endoprothesis component comprising aluminium oxide and zirconium (di)oxide, and being stabilised with e.g. titanium oxide.

EP 1 270 533 A discloses a ceramic bone replacement material comprising a crystalline oxide ceramic such as titanium(IV) oxide dispersed with an amorphous polyanionic intergranular phase.

EP 116 298 A discloses a ceramic bone replacement material consisting of 70-95 mass-% titanium dioxide and the rest hydroxyapatite.

### SUMMARY OF THE INVENTION

It is therefore an object of the present invention to provide a metal oxide scaffold to be used as a medical implant for implantation into a subject that overcome the above mentioned disadvantages, i.e. that have a good biocompatibility and does not cause any adverse reactions when implanted into a subject, which allow for cell growth into the 3-dimensional scaffold and which still has a mechanical stability which allows it to be practically useful as a stabilizing structure. Additionally it is an object of the present invention that the scaffold should have surface properties that result in improved condition for the bone producing cells resulting in faster bone growth on the scaffold surface and subsequently an interconnecting network of bone trabecuale.

The above defined objects are achieved by providing a metal oxide scaffold made of metal oxide comprising titanium oxide wherein the scaffold has a compression strength of about 0.1-150 MPa. More preferably, a metal oxide scaffold of the invention has a compression strength of 5-15 MPa.

In a second aspect, the present invention provides a medical implant comprising such a metal oxide scaffold.

In another aspect the invention relates to a method for producing a metal oxide scaffold comprising the steps of
a) preparing a slurry of metal oxide comprising titanium oxide, said slurry optionally comprising fluoride ions and/or fluorine
b) providing the slurry of step a) to a porous polymer structure
c) allowing the slurry of step b) to solidify
d) removing the porous polymer structure from the solidified metal oxide slurry.

In yet another aspect, the invention relates to the use of a medical implant as disclosed herein for the regeneration, repair, substitution and/or restoration of tissue such as bone, cartilage, cementum and dental tissue.

The invention also relates to the use of a granulated metal oxide scaffold as a bone filling material.

The invention in addition relates to a method for the regeneration, repair, substitution and/or restoration of tissue comprising the implantation into a subject in need thereof of a metal oxide scaffold or a medical implant of the invention.

Since the metal oxide scaffolds of the invention are made of metal oxide comprising titanium oxide which has a good biocompatibility, they may be implanted into a subject without causing adverse reactions, such as allergic reactions. The scaffolds of the invention also have a beneficial effect on the regeneration of tissue due to the material they are made of and their surface structure. The metal oxide scaffolds of the invention in addition have a stability which is particularly suitable for their use in medical implants having enough stability to provide a stabilizing function while still not being to rigid. Such a stability may be achieved by using titanium oxide that is free of contaminations of secondary and/or tertiary phosphates.

### BRIEF DESCRIPTION OF THE DRAWINGS

**Fig. 1****.** Hot plate moulding (HPM). A depression in the plate prevents flattening of the viscous body during the shaping process. The upper part is shaped freely by the combination of internal steam pressure and viscosity.
**Fig. 2****.** Flowchart of the polymer sponge method.
**Fig. 3****.** Struts of a metal oxide scaffold. This strut has not collapsed because of a too short sintering time (A). This picture shows that one wall of the three-sided strut has collapsed removing the former hollow space inside (B).
**Fig. 4****.** Percentage of bone inside the hollow cavity were the scaffold was placed describes the amount of newformed bone. Sham is the control, were the hollow cavity remained empty. Titanium oxide scaffold, titanium oxide scaffold with low flouride and titanium oxide scaffold with medium flouride content had all significantly (p<0.001) increased bone formation inside the scaffold. The error bar displays the standard deviation of the mean and the height of the bar display the mean of measurements (* p<0.001, n=13).
**Fig. 5****.** Trabecular thickness box plot displays the quality of the new formed bone inside the scaffold. Titanium oxide scaffold with low flouride and titanium oxide scaffold with medium flouride content had all significantly (p<0.001) increased bone quality inside the scaffold. The error bar displays the standard deviation of the mean and the height of the bar display the mean of measurements (* p<0.001, ** p<0.05, n= 12).
**Fig. 6****.** EDX mixture ratio of 80wt% TiO₂ and 20 wt% Al₂O₃.
**Fig. 7****.** Vickershardness of TiO₂-Al₂O₃ composite with different mixtures.
**Fig. 8****.** Vickershardness of mixture of ZrO₂ and TiO₂ composites.
**Fig. 9****.** Light microscope image of a sample 60 wt% TiO₂ / 40 wt% ZrO₂. Agglomorates are visible (white regions) and matrix in the grey-black area.

### DEFINITIONS

By ceramics are in the present context meant objects of inorganic powder material treated with heat to form a solidified structure.

"Metal oxide" in the present context relates to an oxide of a metal, such as an oxide of Ti, Zr, Hf, V, Nb, Ta and/or Al. Examples of metal oxides suitable for the present invention include, but is not limited to, TiO₂, Ti₃O, Ti₂O, Ti₃O₂, TiO, Ti₂O₃, Ti₃O₅, ZrO₂, tantalum oxide (e.g. TaO₂) and Al₂O₃ or combinations of these.

"Scaffold" in the present context relates to an open porous structure. A scaffold structure in the present context typically has a combined micro and macro pore size of approximately 10 - 3000 µm, preferably 20-2000 µm, more preferably 30-1500 µm and even more preferably 30-700 µm. Preferably the pore size is above 40 µm, with interconnective pores of at least 20 µm.

By pore diameter thickness is meant the net pore diameter, i.e. the mean net diameter of a pore (without the surrounding walls).

Fractal dimension strut is a statistical quantity that gives an indication of how completely a fractal appears to fill space, as one zooms down to finer and finer scales. There are many specific definitions of fractal dimension and none of them should be treated as the universal one. A value of 1 pertains to a straight line. The higher the number the more complex the surface structure.

Total porosity is in the present context defined as all compartments within a body which is not a material, e.g. the space not occupied by any material. Total porosity involves both closed and open pores.

By inner strut volume is meant the volume of the inner lumen of the strut

Contamination of secondary and/or tertiary phosphate are defined to be present when a slurry as disclosed herein does not respond to the ideal IEP (e.g. if IEP of TiO₂ is higher than 1.7, contamination of the titanium oxide particles is present).

By "sintering" is meant a method for making objects from powder, by heating the material (below its melting point) until its particles adhere to each other. Sintering is traditionally used for manufacturing ceramic objects, and has also found uses in such fields as powder metallurgy.

A "medical implant" in the present context relates to a device intended to be implanted into the body of a vertebrate animal, such as a mammal, e.g. a human mammal. Implants in the present context may be used to replace anatomy and/or restore any function of the body. Examples of implants include, but are not limited to, dental implants and orthopaedic implants. In the present context, the term "orthopedic implant" includes within its scope any device intended to be implanted into the body of a vertebrate animal, in particular a mammal such as a human, for preservation and restoration of the function of the musculoskeletal system, particularly joints and bones, including the alleviation of pain in these structures. In the present context, the term "dental implant" includes within its scope any device intended to be implanted into the oral cavity of a vertebrate animal, in particular a mammal such as a human, in tooth restoration procedures. Dental implants may also be denoted as dental prosthetic devices. Generally, a dental implant is composed of one or several implant parts. For instance, a dental implant usually comprises a dental fixture coupled to secondary implant parts, such as an abutment and/or a dental restoration such as a crown, bridge or denture. However, any device, such as a dental fixture, intended for implantation may alone be referred to as an implant even if other parts are to be connected thereto.

In the present context "subject" relate to any vertebrate animal, such as a bird, reptile, mammal, primate and human.

The mechanical strength of the scaffolds was determined in a compression test (Zwicki, Zwick/Roell, Ulm, Germany) on a load cell of 200 N. The scaffold was to be preloaded with a force of 2 N. The speed of the compression was set to 100 mm/min. Test data were analyzed in testXpert 2.0. DIN EN ISO 3386, 1998.

### DETAILED DESCRIPTION OF THE INVENTION

The present invention relates to metal oxide scaffolds having an improved biocompatibility and a mechanical stability which makes them useful in medical implants. The invention also relates to methods for producing such scaffolds and uses thereof.

Scaffold implants are used in order to regenerate a tissue after loss of tissue volume, as compared to solid implants, which replace a tissue primarily to regain function (e.g. dental implants and hip prosthesis). As most prior art scaffolds are resorbable these are degraded after implantation into a subject. This means that they only have the function of acting as a framework for growth of the regenerated tissue. However, in some instances it is preferred to have a scaffold that in addition to functioning as a framework for cell growth also provides a stabilising function. However, as previously discussed, the previously available scaffolds have problems in terms of low biocompatibility, e.g. causing allergic reactions. Also, it has previously not been possible to produce metal oxide scaffolds comprising titanium oxide which are stable enough to provide a stabilizing function when implanted in a subject while still not being to rigid.

The present inventors have surprisingly found a solution to the problem of being able to provide a scaffold made of a material with good biocompatibility, that also has a stability which makes it practically useful not only for allowing regeneration of tissue, but that also allows the scaffold itself to remain in a subject and provide mechanical stability to the regenerated tissue.

This object is achieved by providing a metal oxide scaffold comprising metal oxide comprising titanium oxide. It is desirable to be able to produce a metal oxide scaffold comprising titanium oxide as titanium oxide is has the ability to osseointegrae, but also to form angionese.

As mentioned above, it has previously not been possible to produce scaffolds comprising titanium oxide that have a stability that is suitable for their use as medical implants. The metal oxide scaffolds comprising titanium oxide of the present invention, however, have a mechanical strength of about 0.1-150 MPa, a strength that is well suited for their use as medical implants. In a preferred embodiment, the mechanical strength is about 5-15 MPa. The compression strength of the scaffolds of the present invention is measured by commonly known methods according to the above.

The invention also relates to a metal oxide scaffold comprising titanium oxide as disclosed herein for the regeneration, repair, substitution and/or restoration of tissue, in particular bone tissue.

Most commercially available titanium oxide powders have surface contaminations of secondary and/or tertiary phosphates. These phosphate contaminations hinder a proper sintering of the metal oxide scaffolds during their preparation and if used for producing titan oxide scaffolds, the resulting scaffolds therefore do not have a satisfactory mechanical strength.

The present inventors have however surprisingly found that by utilizing a titanium oxide powder that is free of contaminations of secondary and/or tertiary phosphates (i.e. containing less than 10 ppm of such contaminations as described later) on the surface of the titanium oxide particles, metal oxide scaffolds comprising titanium oxide can be prepared. Titanium oxide that is free of secondary and/or tertiary phosphates on the surface of the titanium oxide particles may be obtained either by using a titanium oxide powder that already is free from such contaminations (e.g. the titanium oxide from Sachtleben). Alternatively a titanium oxide powder comprising phosphate contaminations may be washed, such as with NaOH (e.g. 1 M) in order to remove the phosphate contaminations.

Titanium oxide scaffolds made of titanium oxide without phosphate contaminations have previously not been produced. Consequently, previously titanium oxide scaffolds having a mechanical strength which make them practically useful have not been able to produce. In the present context, the titanium oxide comprises less than 10 ppm of contaminations of secondary and/or tertiary phosphate. Such titanium oxide is in the present context considered to be free of contaminations of secondary and/or tertiary phosphate.

The strength of the metal oxide scaffolds disclosed herein may be varied by varying the porosity of the scaffolds. A decreasing porosity will increase the strength of the scaffold.

Kim *et al.* (24) disclosed scaffolds of ZrO₂ with a hydroxyapatite (HA) layer. These scaffolds were ascribed the combined advantage of mechanical strength because of the ZrO₂ and a biocompatible surface due to the HA layer. However, in these scaffolds, the HA layer may peel off/wear off during implantation or even crackle and tear and loosen from the surface of the implant due to the difference in mechanical properties between ZrO₂ and Ha when the scaffold is loaded by use. Such HA fragments form small sequestered bodies that effectively initiate a foreign body response that hamper the osseointegration of the scaffold structure. Moreover, another disadvantage of these ZrO₂ scaffolds is that the ZrO₂ surface itself, when exposed by the faults in the HA layer, has little inherent osteoconductive effect, further contributing to the failure of these scaffolds to integrate properly in bone. In comparison, titanium oxide is more osseoinductive than ZrO₂ and as titanium oxide is an integral part of the scaffolds of the invention, there is no risk of loss of this osseoinductive material when the scaffolds are loaded.

An additional advantage with the scaffolds of the invention over prior art scaffolds are a reduced cost for production and an easier production.

Said metal oxide scaffold is intended for implantation into a subject, i.e. as a medical implant, such as a mammal subject, e.g. a human subject. The metal oxide scaffold implants of the invention comprise a porous structure with improved surface properties which enhances their biocompatibility and stimulates the growth of cells and attachment of the implant. The porous structure allows ingrowth of cells into the scaffold, which thereby allows for the regeneration of tissue. The large surface area of the metal oxide scaffolds also facilitates the growth of cells into the structure and thereby the attachment of the scaffold and regeneration of tissue. As the scaffold is made of a material which in itself has a good biocompatibility, adverse reactions to the scaffold when implanted into a subject are reduced.

The present invention provides a macroporous scaffold comprising macropores and interconnections. Macropores of the metal oxide scaffold of the invention are in the range between approximately 10-3000 µm, preferably about 20-2000 µm, more preferably about 30-1500 µm and even more preferably about 30-700 µm. More preferably the macropore diameter is above about 100 µm. Most preferably, the macropore diameter is about 30-700 µm. For bone, the pore size is optimally 30-100 µm. However, it is important that the scaffold also allows for the ingrowth of larger structures such as blood vessels and trabecular bone, i.e. also has pores of about 100 µm or more. It is important that at least some of the pores of the scaffolds of the invention are interconnected.

The pore size may be adjusted by the choice of structure used for producing the scaffold, e.g. the choice of sponge and the number of times this structure is dipped into a slurry comprising the metal oxide (which process is disclosed elsewhere in the text). By altering the pore size one may affect the rate and extent of growth of cells into the scaffold and therefore the constitution of the resulting tissue. In another preferred embodiment the metal oxide scaffold comprises pores which are interconnective or partially interconnective. This means that the pores are not pores with a "dead end" or closed pores, but have at least two open ends allowing for the passage of nutrients and waste products in more then one direction. Thereby, the risk that necrotic tissue forms is reduced. The macroporous system preferably occupies at least 50% volume of the scaffold. The volume of the macro- and micropores in the scaffolds may vary depending on the function of the scaffold. If the aim with a treatment is to replace much bone structure and the scaffold can be kept unloaded during the healing time, the scaffold may be made with a macroporous system occupying up to 90% of the total scaffold volume.

Preferably a metal oxide scaffold according to the invention has a total porosity of about 40-99% preferably 70-90%.

Preferably a metal oxide scaffold according to the invention has a fractal dimension strut of about 2.0-3.0, preferably about 2.2-2.3. The strut thickness affects the strength of the scaffolds, the thicker the struts in the scaffold are, the stronger is the scaffold.

Preferably a metal oxide scaffold according to the invention has an inner strut volume of about 0.001-3.0 µm³, preferably about 0.8-1.2 µm³. A lower volume and a higher fractal number give a stronger scaffold.

It will be understood by those of skill in the art that the surface of the present scaffold of the invention also has a structure on the microlevel and the nanolevel. This micro and nano structure may be modified due to the manufacturing conditions. The pores created by the manufacturing process are on the microlevel in the range of 1-10 µm. The pores on the nanolevel are less than 1 µm in diameter.

A scaffold structure in the present context typically has a combined micro and macro pore size of approximately 10 - 3000 µm, preferably 20-2000 µm, more preferably 30-1500 µm and even more preferably 30-700 µm. Preferably the pore size is above 40 µm, with interconnective pore of at least 20 µm.

As will be obvious later, due to the way the scaffolds are produced, the scaffolds of the invention have a structure of hollow tubules in which the bone will grow and create the interconnecting bone trabeculae. Cells will grow both on the inside and the outside of these tubules.

The size and the shape of the metal oxide scaffold are decided depending on its intended use. By varying the size and shape of the porous structure used when producing the scaffold (see below), the size and shape of the resulting scaffold may be varied. The scaffolds may therefore easily be tailored for their specific use in a specific subject.

The invention in another aspect relates to a metal oxide scaffold comprising titanium oxide that also comprises fluoride and/or fluorine. Such a scaffold is even more stable as the titanium fluoride formed is more chemically stable than titanium oxide in itself (i.e. less soluble from the surface leading to a more stable scaffold being formed). Also, the fluoridation of the scaffold has the further advantage of providing a further improved biocompatibility. This may be due to the fact that the fluoride is assumed to react with the titanium oxide. *In vivo* phosphate from the tissue may in turn replace the fluoride and the phosphate will bind to the titanium in the scaffold. This may induce a bone formation where phosphate in the bone is bound to the titanium implant. Moreover, the released fluoride may catalyze this reaction and induce formation of fluoridated hydroxyapatite and fluoroapatite in the surrounding bone. One embodiment of the invention therefore is a metal oxide scaffold comprising at least one surface which is at least partially covered with fluoride and/or fluorine.

In one embodiment the metal oxide scaffold is treated with an aqueous solution comprising fluoride ions and/or fluorine. For this purpose, preferably an aqueous solution comprising HF, NaF and/or CaF₂ is used. Alternatively, the fluoride/fluorine may be provided via a gas phase and/or as a vapour. The concentration of fluoride and/or fluorine in an aqueous solution is about 0.001-2.0 wt%, preferably 0.05-1.0 wt%. The pH of an aqueous solution comprising fluoride and/or fluorine is preferably approximately pH 0-7, preferably pH 2-6, more preferably pH 2-4.

In another embodiment, the fluoride ions and/or fluorine is provided in the slurry used to prepare the metal oxide scaffold. Thereby, fluoride/fluorine is provided as an integral part of the metal oxide scaffold.

By the fluoridation of the metal oxide scaffolds, an additional increase in their stability is achieved. Thereby, it is by the present invention possible to provide a scaffold that has the advantage of high biocompatibility combined with improved mechanical stability and strength so that the lost bone volume can be replaced by the scaffold and new bone can regenerate and subsequently a new bone structure can be created.

Oxides of titanium to be used in a metal oxide scaffold of the present invention comprises one or more titanium oxides selected from TiO₂, Ti₃O, Ti₂O, Ti₃O₂, TiO, Ti₂O₃, or Ti₃O₅. In one preferred embodiment the titanium oxide in the metal oxide scaffold comprises TiO₂. In one preferred embodiment the metal oxide is titanium oxide comprising one or more titanium oxides selected from Ti₃O, Ti₂O, Ti₃O₂, TiO, Ti₂O₃, or Ti₃O₅ in combination with TiO₂. Preferably, the metal oxides to be used in a metal oxide scaffold of the present invention consists of one or more titanium oxides selected from TiO₂, Ti₃O, Ti₂O, Ti₃O₂, TiO, Ti₂O₃, or Ti₃O₅. In another preferred embodiment the titanium oxide consists of TiO₂.

In addition to titanium oxide, the metal oxide scaffold may further comprise, in mixture, one or more of an oxide of Zr, Hf, V, Nb, Ta and/or Al.

A metal oxide scaffold according to the invention preferably has a titanium oxide content of about 40-100%, more preferably 60-90%, by weight of the metal oxides present in the scaffold.

In another embodiment the metal oxide scaffold comprises ZrO₂. In this embodiment preferably approximately 90% or more of said metal oxide is ZrO₂.

In another embodiment the metal oxide scaffold comprises ZrO₂ in combination with one or more of MgO, CaO, or Y₂O₃. This will improve the mechanical properties of the scaffold.

Another embodiment relates to a metal oxide scaffold, wherein said metal oxide comprises Al₂O₃. In this embodiment preferably 90% or more of said metal oxide is Al₂O₃.

In yet another preferred embodiment of the invention the metal oxide scaffold comprises a composite of Al₂O₃ and TiO₂. A composite may also be comprised of TiO₂ and ZrO₂. Such a scaffold will have an increased strength.

A metal oxide scaffold according to the present invention may also comprise particles of metal oxide (of size ca 10 nm-100 µm) that are mixed with the metal oxide slurry during preparation of the metal oxide scaffolds. A scaffold prepared in this way, comprising particles of metal oxide interspersed in a metal oxide, has an increased stability.

The metal oxide scaffolds of the invention may also optionally comprise CaPO₄, Cl⁻, F⁻ and/or carbonate. Such additives may even further increase the biocompatibility of the scaffolds and improve their osseointegration.

In another aspect, the present invention relates to a medical implant comprising a metal oxide scaffold. A medical implant according to the present invention may be a metal oxide scaffold in any embodiment of the invention in itself. Alternatively, the medical implant may comprise a metal oxide scaffold of the invention in combination with another structure, such as orthopedic, dental or any other fixating devices or implants. The invention also relates to a medical implant comprising a metal oxide scaffold comprising titanium oxide for the regeneration, repair, substitution and/or restoration of tissue, in particular bone tissue.

In another aspect the invention relates to a slurry for preparing a metal oxide scaffold comprising titanium oxide. This slurry comprises an aqueous solution of titanium oxide powder, said titanium oxide powder comprising less than about 10 ppm of contaminations of secondary and/or tertiary phosphates. The aqueous solution comprises regular or deionized water as a solvent. The ratio of titanium oxide powder compared to water content (by weight) is at least 2:1. The slurry may also comprise other metal oxides as disclosed herein. Optionally the slurry may also comprise fluoride ions or fluoride (e.g. in a concentration of 0.01 wt% HF). Optionally this slurry may also comprise other constituents such as CaPO₄, Cl⁻, F⁻, carbonate, MgO, CaO, and/or Y₂O₃, that are of interest to have in the metal oxide scaffold. The slurry is preferably an aqueous slurry and withstands temperatures of 500°C or more.

The titanium oxide powder used for preparing the slurry may be in the amorphous, anatase, brookit or rutile crystal phase.

It is advantageous that the particle size of the metal oxides in the slurry is as small as possible in order to ensure a proper sintering (see below for details on the sintering process). Thus, the metal oxide particles need to be reduced to smaller pieces and evenly distributed prior to sintering. By using titanium oxide containing less than about 10 ppm of contaminations of secondary and/or tertiary phosphates when preparing the slurry, the titanium oxide particles are small enough to allow a proper sintering without the addition of organic antiagglomerating compounds. This results in an easier production process. A preferred embodiment of the present invention therefore relates to a slurry comprising titanium oxide to which no additives, such as organic additives, and/or surfactants have been added. However, if other metal oxides but titanium oxide are to be present in the scaffold it may be necessary to add organic additives such as binders e.g. polysaccharide binders (e.g. Product KB 1013, Zschimmer & Schwarz GmbH, Lahnstein, Germany) in order to reduce agglomerations and to stabilize the slurry.

A typical slurry used in the present invention comprises water, metal oxide particle and optionally organic parts such as binders and/or surfactants to reduce surface tension.

One preferred embodiment relates to a metal oxide scaffold comprising TiO₂ having less than about 10 ppm of contaminations of secondary and/or tertiary phosphates. Another preferred embodiment relates to a metal oxide scaffold containing only TiO₂ containing less than about 10 ppm of contaminations of secondary and/or tertiary phosphates as the metal oxide in the scaffold.

The slurry for preparing a metal oxide scaffold comprising titanium oxide has a pH value of about 1.0 to 4.0, preferably about 1.5-2.0. It is preferable to reduce the size of the titanium oxide particles as close as possible to the pH value which gives the theoretical isolectric point of titanium oxide. For TiO₂ this pH value is 1.7. The mean particle size of the titanium oxide particles is preferably 10 µm or less, more preferably 1.4 µm or less. Preferably the titanium oxide particles are monodispersed.

When producing the metal oxide scaffolds of the present invention, it is important that the starting slurry comprising the metal oxide(s) is aqueous and withstands burning at temperatures from 500°C and above.

A method for producing a metal oxide scaffold of the invention comprises the steps of:
a) preparing a slurry of metal oxide comprising titanium oxide, said slurry optionally comprising fluoride ions and/or fluorine
b) providing the slurry of step a) to a porous polymer structure
c) allowing the slurry of step b) to solidify
d) removing the porous polymer structure from the solidified metal oxide slurry.

The details of composition of the slurry used in this process is described elsewhere in this text.

The slurry is prepared by dispersing the metal oxides and other optional constituents in the solvent used. Preferably, the metal oxides are gradually added to the solvent while stirring and readjusting the pH with e.g. 1 M HCl to keep it at the preferred pH value. The slurry is then e.g. further dispersed with a rotational dispermat with metal blades, preferably titanium blades. Preferably this is performed at a speed of at least 4000 rpm and for at least 4 hours. More preferably this step is performed at 5000 rpm for 5 hours or longer. The pH of the slurry is regularly adjusted to the chosen pH value.

The slurry is then provided to a porous polymer structure. The porous polymer structure may e.g. be a sponge structure, such as a synthetic sponge. The material the porous polymer structure is made of is preferably an organic material in order to facilitate the removal of the porous polymer structure from the scaffold by combustion. The porous polymer structure is therefore an organic sponge structure, preferably an organic porous polymer sponge, preferably a polyethylene, silicone, celluloses or polyvinylchloride sponge. One example of a preferred porous polymer structure is a 45 or 60 ppi Bulbren polyurethane foam (Bulbren S, Eurofoam GmbH, Wiesbaden, Germany). The porous polymer structure is preferably washed with water before providing the slurry thereto in order to remove residuals and/or contaminations. The slurry may be provided to the porous polymer structure by immersing the porous polymer structure in the slurry. After the immersion excess slurry may be removed by squeezing and/or centrifuging the porous polymer structure immersed in the slurry. The slurry is then allowed to solidify on the porous polymer structure, e.g. by drying the porous polymer structure immersed in the slurry for at least 5 hours and more preferably for about 24 hours. By varying the number of times steps b)-d) are performed, the size of the macropores of the scaffold may be varied.

The size and shape of the metal oxide scaffold produced may be adjusted by adjusting the size and shape of the porous polymer structure used. Thereby it is possible to produce a scaffold that is tailor-made for a specific intended implantation site of a specific subject.

The next step in the preparation of the metal oxide scaffolds is to remove the porous polymer structure from the thereon solidified slurry to obtain the scaffold structure.

In a preferred method of the invention, step d) is performed by removing the porous polymer structure from the solidified metal oxide slurry by heating.

Preferably the porous polymer structure is a combustible structure. Thereby step d) in the above method may e.g. be performed by burning off the porous polymer structure from the solidified metal oxide slurry. The temperature and time necessary to perform this process will, as the skilled person readily understands, depend on the material that the porous polymer structure is made of. Importantly, the temperature and time should be selected to allow for more or less complete removal of the porous polymer structure. The skilled person will know how to select the necessary time and temperature for a specific porous polymer structure and scaffold to achieve this.

In a preferred method step d) is performed by
i) slow sintering of the porous polymer structure with the solidified metal oxide slurry to about 500°C and holding this temperature for at least 30 minutes,
ii) fast sintering to about minimum 1500°C or to about 1750°C at ca 3 K/min and holding this temperature for at least 10 hours, and
iii) fast cooling to room temperature at least 3 K/min.

The biological response to the metal oxide scaffolds may be varied in different ways. One example is to vary the crystal phase of the coated layer of titanium oxide. The crystal phase can be altered by ending the sintering of the coated layer at different temperature. Sintering above 960±20°C gives the rutile phase, while sintering below 915±20°C gives the anatase phase.

Finally the surface of the resulting scaffold may be modified e.g. by providing fluoride ions and/or fluoride thereon. The fluoride and/or fluorine may be provided in an aqueous solution comprising HF, NaF and/or CaF₂, wherein the concentration of fluoride and/or fluorine in the solution is approximately 0.001-2.0 wt%, preferably 0.05-1 wt% and the fluoride and/or fluorine is provided to the surface of the scaffold by immersing the scaffold in this solution. Ideally this is not performed longer than 120 seconds in a solution with 0.1 wt% fluoride and/or fluorine. Alternatively, the fluoride/fluorine may be provided via a gas phase and/or as a vapour.

Also, biomolecules may be provided to the surface of the scaffolds. If biomolecules are to be provided to the metal oxide scaffold, these may be provided after step d) of the method above. The presence of biomolecules may further increase the biocompatibility of the scaffolds and rate of cell growth and attachment. Biomolecules comprise in the present context a wide variety of biologically active molecules including natural biomolecules (i.e. naturally occurring molecules derived from natural sources), synthetic biomolecules (i.e. naturally occurring biomolecules that are synthetically prepared and non-naturally occurring molecules or forms of molecules prepared synthetically) or recombinant biomolecules (prepared through the use of recombinant techniques). Examples of biomolecules of interest include, but are not limited to biomolecules disclosed in US 2006/0155384, such as bioadhesives, cell attachment factors, biopolymers, blood proteins, enzymes, extracellular matrix proteins and biomolecules, growth factors and hormones, nucleic acids (DNA and RNA), receptors, synthetic biomolecules, vitamins, drugs biologically active ions marker biomolecules etc., including proteins and peptides such as statins and proteins or peptides that stimulate biomineralization and bone formation. The biomolecules may e.g. be attached to the surface of the scaffold via dipping into a solution comprising the biomolecule or via an electrochemical process, such processes being known by the skilled person.

In another aspect the invention relates to a metal oxide scaffold as disclosed herein for use as a medical implant. One embodiment relates to a medical implant for use for the regeneration of tissue. Another embodiment relates to a medical implant for use for the regeneration of bone.

In another aspect the invention relates to the use of a metal oxide scaffold as disclosed herein for the preparation of a medical implant for the regeneration, repair, substitution and/or restoration of a tissue, such a bone tissue.

The metal oxide scaffolds of the invention may also be granulated and used as a bone filling material. The advantage of using granulated scaffolds as filling material is that it may be used to fill bone void (pockets with no bone, where the scaffold may be granulated to fill all the empty volume). Therefore, in another aspect the invention relates to a granulated bone filling material comprising a metal oxide scaffold according to the invention. For this purpose, the crushed metal oxide scaffold particles may be sorted by particle size. For filling bone cavities, the particle size is optimally in the range of 0.05-5 mm (mean diameter), preferably 0.1-2 mm, most preferably 0.2-1 mm. Particles of the same size or mixtures of different sizes may be used.

In another aspect the invention relates to a metal scaffold as disclosed herein, which has been granulated, for use as a bone filling material. In yet another aspect the invention relates to the use of a metal oxide scaffold as disclosed herein which has been granulated to the preparation of a bone filling material.

In yet another aspect the invention relates to a method for the regeneration, repair, substitution and/or restoration of tissue comprising the implantation into a subject in need thereof of a metal oxide scaffold or a medical implant as disclosed herein.

Tissue engineering involves the development of a new generation of biomaterials capable of specific interactions with biological tissues to yield functional tissue equivalents. The underlying concept is that cells can be isolated from a patient, expanded in cell culture and seeded onto a scaffold prepared from a specific biomaterial to form a scaffold/biological composite called a "TE construct". The construct can then be grafted into the same patient to function as a replacement tissue. Some such systems are useful for organ tissue replacement where there is a limited availability of donor organs or where, in some cases (e.g. young patients) inadequate natural replacements are available. The scaffold itself may act as a delivery vehicle for biologically active moieties from growth factors, genes and drugs. This revolutionary approach to surgery has extensive applications with benefits to both patient well-being and the advancement of health care systems.

The scaffolds of the invention may be implanted into a subject wherein cells will grow into the scaffold structure. It is also possible to seed and grow cells on the implant prior to implantation. The novel interconnected macroporous structure of the present metal oxide scaffold is especially suitable for tissue engineering, and notably bone tissue engineering, an intriguing alternative to currently available bone repair therapies. In this regard, bone marrow-derived cell seeding of the metal oxide scaffold is performed using conventional methods, which are well known to those of skill in the art (as described in Maniatopoulos et al, in Cell Tissue Res 254, 317-330, 1988). Cells are seeded onto the metal oxide scaffold and cultured under suitable growth conditions. The cultures are fed with media appropriate to establish the growth thereof.

As set out above, cells of various types can be grown throughout the present metal oxide scaffold. More precisely, cell types include hematopoietic or mesenchymal stem cells, and also includes cells yielding cardiovascular, muscular, or any connective tissue. Cells may be of human or other animal origin. However, the metal oxide scaffold of the present invention is particularly suited for the growth of osteogenic cells, especially cells that elaborate bone matrix. For tissue engineering, the cells may be of any origin. The cells are advantageously of human origin. The present method of growing cells in a three dimensional metal oxide scaffold according to the invention allows seeded osteogenic cells, for example, to penetrate the metal oxide scaffold to elaborate bone matrix, during the *in vitro* stage, with pervasive distribution in the structure of the metal oxide scaffol. Osteogenic cell penetration and, as a result, bone matrix elaboration can be enhanced by mechanical, ultrasonic, electric field or electronic means

The scaffolds of the present invention are useful whenever one is in need of a structure to act as a framework for growth of cells, such as for regeneration of a tissue. The scaffolds of the invention are particularly useful for the regeneration of bone and cartilage structures. Examples of situations where the regeneration of such structures may be necessary include trauma, surgical removal of bone or teeth or in connection to cancer therapy. The invention in another aspect therefore also relates to the use of a medical implant as disclosed herein for the regeneration of tissue, such as bone, cartilage, cementum and dental tissue and a method for regenerating such tissues comprising the implantation of a such a medical implant into a subject in need thereof.

Examples of structures in a subject which wholly or partially may be replaced include, but are not limited to, Cranio-facial bones, including arcus zygomaticus, Bones of the inner ear (in particular the malleus, stapes and incus, maxillar and mandibular dentoalveolar ridge, walls and floor of eye sockets, walls and floor of sinuses, skull bones and defects in skull bones, socket of hip joint (Fossa acetabuli), e.g. in the case of hip joint dysplasias, complicated fractures of long bones including (but not restricted to) humerus, radius, ulna, femur, tibia and fibula, vertebrae, bones of the hands and feet, finger and toe bones, filling of extraction sockets (from tooth extractions), repair of periodontal defects and repair of periimplant defects.

In addition the scaffolds of the present invention are useful for the filling of all types of bone defects resulting from (the removal of) tumors, cancer, infections, trauma, surgery, congenital malformations, hereditary conditions, metabolic diseases (e.g. osteoporosis and diabetes).

### EXPERIMENTAL SECTION

### EXAMPLE 1: TIO₂ SLURRY RECIPE

The used slurry consists of an electrostatically stabilized TiO₂-suspension without further additives. The components of the slurry are deionised water, TiO₂-Powder (batch 1170117, Sachtleben Hombitan Anatase FF-Pharma, Duisburg, Germany) and 1 mol/l HCl (Merck Titrisol, Oslo, Norway). The suspension was set at pH 2.2 by the HCl. In order to achieve an optimal dispersion, the suspensions in the agitator mill, with the ceramic(s) double meal disk were prepared. As grinding bodies 600 g Zirconox CE milling beads (Jyoti GmBH, Drebber, Germany) were used, with a diameter of 0.4-0.7 mm. The production of the slurry was done in the following manners:
1. 118.8 g deionised water with 1.2 g 1 mol/l HCl is placed in a water-cooled container with milling beads.
2. The addition of that the total amount of 210 g TiO₂-Powder takes place gradually. The TiO₂-Pulver is added into the liquid stepwise with a stirring speed at about 1000 RPM. The suspension's viscosity rises slowly. This behaviour is to be attributed to the fact that protons from the liquid deposit themselves to the OH groups of the TiO₂ of powder. Thus the pH value in the suspension shifts to pH 4.2. In order to lower the viscosity again, with a pipette 1 M HF is titrated into the suspension. Usually 1 ml HF is sufficient. The viscosity is thus controlled with the additional HCl into the slurry.
3. The HCl titration continues until the total amount of 210 g TiO₂ is completely dispersed. Altogether about 8 mL of 1 M HCl is usually needed for this amount. Thus the solid content in the suspension amounts to 30 Vol. %.
4. After this dispersion the agglomerates and aggregates are destroyed by milling at higher speed of 4000 RPM. After approximately 5-6 h an optimal result is obtained.
5. The viscosity of the suspension can be strongly affected depending upon application purpose by change of the solid content or the pH value. Higher viscosities are aimed for the production of thicker scaffolds, whereas thinner and finer structures need lower viscosity.
6. Small gas bubbles may interfere with the ceramic production and should thus be dismissed. This can be done by e.g. rotational evaporation or ultrasonic bath. In addition the slurry could be filtered with nets of pore size of 100 µm and 50 µm. This procedure also reduces the inhomogeneities.
7. There are several means to produce a scaffold after having obtained the proper ceramic slurry. The following two sections describe two methods, hot plate moulding reported by Eckardt et al [12] and the polymer sponge method has be described by Haugen et al [13].

### EXAMPLE 2: TIO₂ SLURRY RECIPE WITH FLUORIDE DOPING

The used slurry consists of an electrostatically stabilized TiO₂-suspension without further additives. The components of the slurry are deionised water, TiO₂-Powder (batch 1170117, Sachtleben Hombitan Anatase FF-Pharma, Duisburg, Germany) and 1 mol/l HCl (Merck Titrisol, Oslo, Norway). The suspension was set at pH 2.2 by the HCl. In order to achieve an optimal dispersion, the suspensions in the agitator mill, with the ceramic(s) double meal disk were prepared. As grinding bodies 600 g Zirconox CE milling beads (Jyoti GmBH, Drebber, Germany) were used, with a diameter of 0.4-0.7 mm. The production of the slurry was done in the following manners:
1. 118.8 g deionised water with 1.2 g 1 mol/l HCl is placed in a water-cooled container with milling beads.
2. The addition of that the total amount of 210 g TiO₂-powder takes place gradually. The TiO₂-powder is added into the liquid stepwise with a stirring speed at about 1000 RPM. The suspension's viscosity rises slowly. This behaviour is to be attributed to the fact that protons from the liquid deposit themselves to the OH groups of the TiO₂ of powder. Thus the pH value in the suspension shifts to pH 4.2. In order to lower the viscosity again, with a pipette 1 M HF is titrated into the suspension. Usually 1 ml HF is sufficient. The viscosity is thus controlled with the additional HCl into the slurry.
3. Adding HF acid into the slurry also has the effect to dope the TiO₂ into titanium-fluoride containing compounds.
4. The HF titration continues until the total amount of 210 g TiO₂ is completely dispersed. Altogether about 8 mL of 1 M HF is usually needed for this amount. Thus the solid content in the suspension amounts to 30 Vol. %.
5. The amount of doping is controlled by the amount of HF added to the slurry, and can be replace by 1 M HCl to lower the fluoride concentration.
6. After this dispersion the agglomerates and aggregates are destroyed by milling at higher speed of 4000 RPM. After approximately 5-6 h an optimal result is obtained.
7. The viscosity of the suspension can be strongly affected depending upon application purpose by change of the solid content or the pH value. Higher viscosities are aimed for the production of thicker scaffolds, whereas thinner and finer structures need lower viscosity.
8. Small gas bubbles may interfere with the ceramic production and should thus be dismissed. This can be done by e.g. rotational evaporation or ultrasonic bath. In addition the slurry could be filtered with nets of pore size of 100 µm and 50 µm. This procedure also reduces the inhomogeneities.

### EXAMPLE 3: SCAFFOLD PRODUCTION-HOT PLATE MOULDING

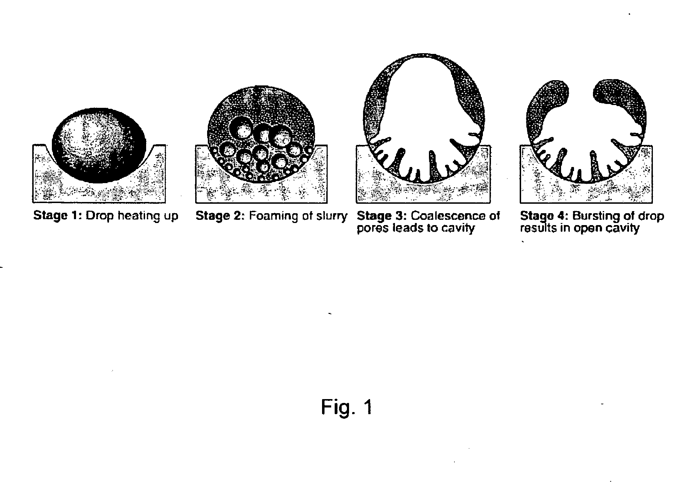

Hot plate moulding is a shaping process which utilises the effect of steam to mould a drop of suspension containing ceramic raw materials into hollow-sphere geometry (Fig. 1). The fluoride-doped TiO₂ slurry was prepared (see Example 2) mixed with 6.0 g polyethylene-copolymer powder (Terpolymer 3580, Plastlabor, Switzerland) and 5.0 g phenolic resin powder (FP 226, Bakelite, Germany). A brass plate with spherical depressions was heated to 320°C. Using a 2 ml syringe, a drop of slurry was then given into each depression. Heat transfer from the plate to the drop led to release of steam which then formed bubbles within the drop. Coalescence of these steam bubbles created a large cavity in the centre of the drop, and progressive drying prevented the structures from collapsing. The continuously rising steam pressure finally led to bursting of the still viscous, topmost part of the drop. Surface tension acted to round the rims of this opening. Further, heat transfer from the hot plate melted and pyrolysed the polymer powders in the formed body, thus stabilising the hollow sphere shape and allowing releasing them from the hot plate. The polymers were removed by heating to 800°C within 5 h. The pre-fired samples were sintered in an electrical furnace for 15 min at 1620°C resulting in a porous ceramic [12].

### EXAMPLE 4: SCAFFOLD PRODUCTION-POLYMER SPONGE METHOD

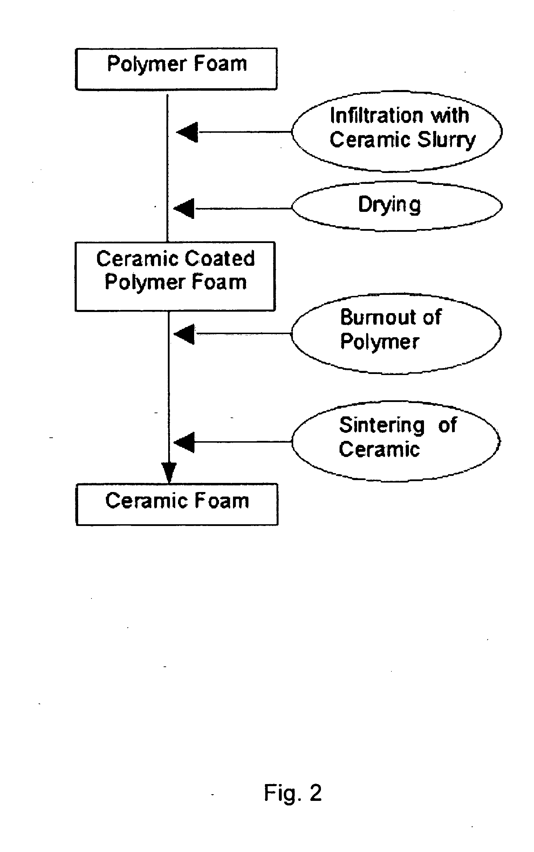

Reticulated open-pore ceramics are produced via the replication of a polymeric porous structure. The patent on this technique called the "replication" or "polymer-sponge" method was first filed by Schwartzwalder and Somers in 1963 [14]. It is the standard method for producing alumina, zirconium, silicon carbide and other ceramic foams [15-19]. The foams are manufactured by coating polyurethane foam fluoride-doped TiO₂ slurry was prepared (see Example 2). The polymer, having already the desired macrostructure, simply serves as a sacrificial scaffold for the ceramic coating. The slurry infiltrates the structure and adheres to the surface of the polymer. Excess slurry is squeezed out leaving a ceramic coating on the foam struts. After drying, the polymer is slowly burned away in order to minimize damage to the porous coating. Once the polymer has been removed, the ceramic is sintered to the desired density. The process replicates the macrostructure of the polymer, and results in a rather distinctive microstructure within the struts. A flowchart of the process is given in (Fig. 2).

### EXAMPLE 5: SCAFFOLD PRODUCTION - POLYMER SPONGE METHOD WITH COATING

Reticulated open-pore ceramics are produced via the replication of a polymeric porous structure. The patent on this technique called the "replication" or "polymer-sponge" method was first filed by Schwartzwalder and Somers in 1963 [14]. It is the standard method for producing alumina, zirconium, silicon carbide and other ceramic foams [15-19]. The foams are manufactured by coating polyurethane foam with the TiO₂ slurry prepared (see Example 1). The polymer, having already the desired macrostructure, simply serves as a sacrificial scaffold for the ceramic coating. The slurry infiltrates the structure and adheres to the surface of the polymer. Excess slurry is squeezed out leaving a ceramic coating on the foam struts. After drying, the polymer is slowly burned away in order to minimize damage to the porous coating. Once the polymer has been removed, the ceramic is sintered to the desired density. The foam is now dipped into the fluoride-doped TiO₂ slurry which is described in Example 2, and subsequently dried and sintered. The dipping procedure continues until an even coating of fluoride-doped TiO₂ covers the TiO₂ scaffold.

### EXAMPLE 6: SLURRY RECIPE

The used slurry consists of a water-based electrostatically stabilised TiO₂-suspension without any further organic additives. The components of the slurry are sterilised water, TiO₂ powder (Pharma FP Hobitam, Sachtleben GmbH, Duisburg, Germany) and 1 mol/L HCl (Merck, Oslo, Norway). The suspension was set at pH 1.7 by HCl. In order to achieve optimal particle sizes, the ceramic slurry was dispersed with a high speed dissolver (Dispermat Ca-40, VMA-Getzmann GmbH, Reichshof , Germany) with a custom made titanium rotorblade. The production of slurry was done in the following manners:
1. Adding 72 grams of TiO₂ powder gradually added into 29.7 ml deionised water at pH 1.7 in a water cooled container under stirring at a low-rotation speed 1000 rpm.
2. The TiO₂ powder had to be added gradually while the temperature was held between 20°C and 25°C. The temperature was decreased to 10°C -18°C when the rotation speed was increased to 4000 rpm.
3. When the slurry was homogenous, the rotation speed was increased to 5000 rpm for 5 hours.
4. The pH was controlled every 30 min and readjusted to pH 1.7 by 1 mol/L HCl.

### EXAMPLE 7: SCAFFOLD PRODUCTION - POLYMER SPONGE, 2nd EXAMPLE

Reticulated open-pore ceramics are produced via the replication of a polymeric sponge structure. This method is referred to as the "schwartzwald process" or the polymer sponge method [14]. Fully reticulated polyester based polyurethane foams with 60 ppi (Bulbren S, Eurofoam GmbH, Wiesbaden, Germany) were used in this study. The foams were supplied in large plates 8 mm in thickness and were cut to size by punching them out with a metal stamp to cylinders of 12 mm in diameter. The tablets were then washed in 1 I deionised H₂O and 10 ml Deconex (Burer Chemie AG, Zuchwill, Switzerland) for two minutes, and subsequeritly in ethanol (Absolute, Arcus, Oslo, Norway). The tablets were then dried at room temperature for 24 h and stored in PE-bags. The slurry was prepared as described in Example 6. The polymer foams were then dipped into this ceramic slurry. These foams were then later were centrifuged (Biofuge 22R Heraeus Sepatech, Osterode, Germany) at 1500 rpm for two minutes at 18°C. The samples were then placed onto a porous ceramic plate and dried at room temperature for at least 24 h prior to sintering. The heating schedule for the burnout of the polymer and the sintering of the ceramic part was chosen as follows: Slowly heating to 450 °C with 0.5 K/min, 1 h holding time at 450 °C, heating to 1500 °C with 3 K/min, 50 h holding time, cooling to room temperature with 6 K/min (HTC-08/16, Nabertherm GmbH, Bremen, Germany).

**Table 1**

| **Sequence** | **Temperature range (C°)** | **Heating rate (K/min)** | **Duration (min)** |
|---|---|---|---|
| 1 | 25-450 | 0.5 | 850 |
| 2 | 450 | 0 | 60 |
| 3 | 450-1500 | 3 | 350 |
| 4 | 1500 | 0 | 3000 |
| 5 | 1500-50 | 6 | 242 |

### Sintering procedure

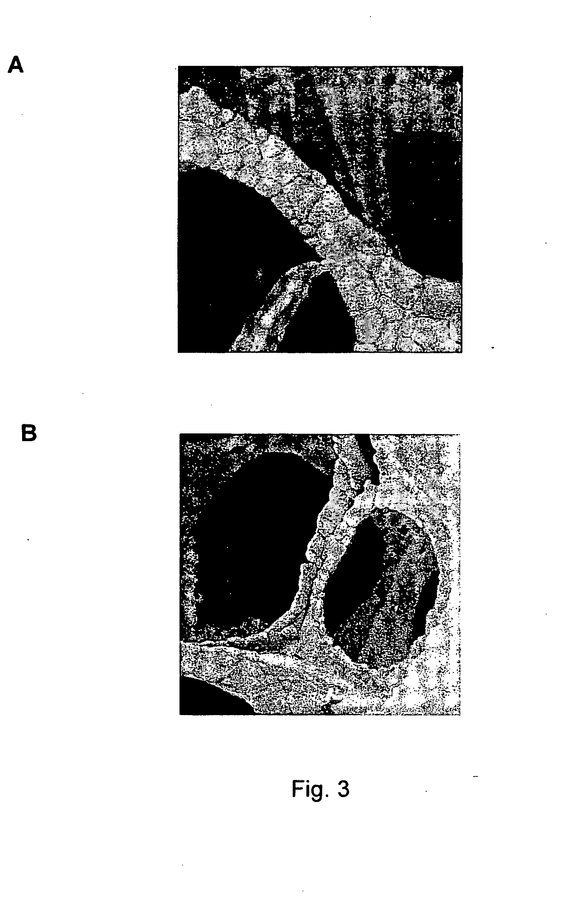

Collapsing of the ceramic struts was reached when maximum temperature of 1500°C was achieved and when this temperature was held for more than twenty hours. While energy was transferred to the scaffolds the hollow space made by burning out the polymer foam collapsed, and a tighter and much stronger structure was made (Fig. 3).

The sintering process produced nanostructure surface on the fused TiO₂ particle. The grain boundary, where the fusion has occurred is visible. At higher magnifications a wavelike nano-structured surface is visible (figure not shown).

The structure on the sintered titanium oxide grain had the following roughness parameters presented in Table 2.

**Table 2. Surface topography and morphology of the titanium oxide scaffolds (n=5). Roughness parameters of the titanium oxide scaffold surface applied to single grains.**

| | Sa | Sq | Ssk | Sku | Sfd (1) | Sci |
|---|---|---|---|---|---|---|
| | µm | µm | <no unit> | <no unit> | <no unit> | <no unit> |
| AVERAGE | 0.89 | 1.12 | -0.87 | 3.28 | 2.35 | 0.53 |
| Standard deviation | 0.42 | 0.56 | 0.31 | 0.79 | 0.06 | 0.26 |

The surface roughness, Sa, on the titanium oxide scaffolds were 890 nm, and the rooth mean square, Sq, 1.12 µm. Since the skewness, Ssk, is slightly negative, there are more surface with valley than peaks. The kurtosis reveal that the peaks are steep. The fractal number, Sfd, shows that the surface is complex and the fluid retention number, Sci, is with a range which has been positively correlated with bone attachment.

Typically Sa is 0.3-1.1 µm, Sq is 0.4-1.4 µm, Ssk -1.2 til 1.2, Sku is 1-4 and Sci is 0.2-1.5 µm. Preferably Sa is about 0.9. Preferably Sq is about 1.2. Preferably Ssk is -0.9. Preferably Sku is 3. Preferably Sci is 0.63.

### Characterisation of titanium oxide scaffold doped with fluoride

### Pore structure

This structure is similar to described TiO₂ scaffolds.

### EXAMPLE 8: SCAFFOLD PRODCTION - POLYMER SPONGE, DOUBLE COATING

The scaffolds were made and sintered as described in Example 7. These scaffolds were then dipped into the slurry as described in Example 6 the and later centrifuged (Biofuge 22R Heraeus Sepatech, Osterode, Germany) at 1500 rpm for two minutes at 18°C. The samples were then placed onto a porous ceramic plate and dried at room temperature for at least 24 h prior to sintering. The second sintering stage was performed to 1500 °C at a rate of 3 K/min, and held at 1500°C for 30 hrs.

### EXEMPLE 9: SCAFFOLD PRODUCTION - POLYMER SPONGE, DOUBLE COATING DOPED WITH FLUORIDE

Scaffolds were prepared as described in Example 8. Then the scaffolds were dipped in 0.2 wt% of hydrofluoric acid. 20 scaffolds were dipped for 90 sec and 20 scaffolds were dipped for 120 sec. Thereafter the scaffolds were rinsed for 1 minute in sterile water, air dried in a laminar flow and packed in sterile packaging.

### EXAMPLE 10: CHARACTERISATION OF TITANIUM OXIDE SCAFFOLD PRODUCED ACCORDING TO EXAMPLES 8 AND 9

The scaffolds (n=49) were analysed using a Skyscan1072 (Skyscan, Aartselaar, Belgium) desktop X-ray CT scanner at 7 µm voxel resolution (50× magnification), X-ray tube current 173 µA and voltage 60 kV with a 0.5 mm aluminum filter. Specimens were mounted vertically on a plastic support and rotated through 180° around the long axis (z-axis) of the sample. Four absorption images were recorded every 0.300° of rotation. These projection radiographs were used in standard cone-beam reconstruction software to generate a series of 1024 8-bit axial slices, each of 1024×1024 pixels, that had Z-dimensional spacing equal to the within slice pixel spacing. The resulting 3-D data sets were hence isotropic with voxel spacing of 7 µm over the entire 1024³ spatial range. 3-D reconstruction of the internal pore morphology was carried out using these axial bitmap images and analysed by CTan and CTvol (Skyscan, Aartselaar, Belgium), where the grey scale threshold was set between 55 and 230. Additional noise was removed by the function "despeckling". All white object smaller than 50 voxels were thus removed prior to further analysis. All images underwent a 3D analysis, following by a "shrink-wrap" function, which allowed measuring the volume of the hollow struts. The density of the scaffold strut was measured using the same software, and calibration was taken at 1.25 and 1.75 g/cm3. The calibration from grayscale to density was performed to Hounsfield unit correction. The relationship was assumed linear.

The BET Surface Area was found to be 5.0664 m²/g , when analysing with liquid nitrogen (TriStar 3000, Micromeretics, Mönchengladbach, Germany)

### IN VIVO EXPERIMENT

Three type of scaffolds were tested as described in example 8 and 9. Double coated titanium oxide scaffolds are referred in this text to as TiO₂. Double coated titanium oxide scaffolds dipped in 0.2 wt.% HF for 90 sec are referred in this text to as TOS. Double coated titanium oxide scaffolds dipped in 0.2 wt.% HF for 120 sec are referred in this text to as THF.

### Animals and surgical procedure

Eighteen (18) New Zealand White female rabbits, 6 months old and 3.0-3.5 kg, were used in the study (ESF Produkter Estuna AB, Norrtälje, Sweden). The animals were kept in cages during the experimental period. Room temperature was regulated to 19 ± 1 °C and humidity was 55 ± 10 %.

The experiments had been approved by the Norwegian Animal Research Authority (NARA) and registered by this authority. The procedures have thus been conducted in accordance with the Animal Welfare Act of December 20th 1974, No 73, Chapter VI, Sections 20-22 and the Regulation on Animal Experimentation of January 15^{th} 1996.

The rabbits were sedated by injection with 0.05-0.1 ml/kg s.c. fluanisone/fentanyl (Hypnorm®, Janssen, Belgium) and 2 mg/kg bw i.v. Midazolam (Dormicum®, Roche, Switzerland) ten minutes prior to removal from the cages. With any signs of waking up, diluted Hypnorm® was injected slowly i.v. until adequate effect was achieved. Lidocain/adrenalin (Xylocain/Adrenalin®, AstraTech AB, Mölndal, Sweden) 1.8-ml s.p. was administered locally at the operation site. Before surgery the operation sites were depilated and washed with soft soap. Animals were placed on their back on the operation table, covered with sterile cloths and the operating sites were disinfected with Chlorhexidingluconat 5 mg/ml (Klorhexidin, Galderma Nordic AB, Sweden).

An incision was made on the proximal-anterior part of tibiae, penetrating all soft tissue layers. The periosteum was elevated and retained by a self-retaining retractor. Four guide holes were made with a twist drill (Medicon® CMS, Germany) using a drill guide to ensure standardised and correct positioning. A custom made stainless steel bur (diameter 6.25 mm) mounted in a slow-speed dental implant drill was used for making a platform for titanium implants. With another drill (diameter 3 mm) fixated in the central guide hole, was used to removed the centrally bone, leaving the bone marrow exposed. Scaffolds (as described in example 8 and 9) were then placed into the bone marrow as follows: one leg with one control TiO2 (T) and one empty defect (SHAM), and in the other leg two fluoride-doped scaffolds (TOS or THF), to have internal controls in the same animal. TOS and THF sample were not place together. All grinding of the bone was done with copious physiological saline solution irrigation. A coin-shaped machined titanium implant (6.25 mm diameter and 1.95 mm) covered with a teflon cap and a pre-shaped titanium maxillofacial bone plate (medicos® CMS, Germany) retained with two titanium screws (Medicon® CMS, Germany) was situated on the cortical bone as stabilizer for the scaffold. The soft tissue was repositioned and sutured with Dexon®II, (Sherwood-Davis & Geck, UK). Following surgery, each animal received a s.c. injection with 20 ml NaCl warmed to body temperature and buprenorphin (Temgesic ®, Reckitt & Colman, England) 0.02 - 0.05 mg/kg s.c. A second injection of Temgesic, also 0.05 mg/kg s.c. was given at least 3 hours after the first/previous Temgesic injection. Health condition was monitored during the study period and the operation sites were carefully examined daily until wound healing was complete. After a healing period of eight weeks, rabbits were euthanised using fluanison/fentanyl (Hypnorm®, Janssen, Belgium) 1.0 ml i.v. followed by pentobarbital (Mebumal®, Rikshospitalets Apotek, Norway) 1 ml/kg body weight i.v. Immediately after euthanisation, an incision was made through the soft tissue on the tibial bone. The titanium plate covering the implants was exposed and removed. A hole was made in the centre of the PTFE cap with a hollow needle, and pressurised air was applied to remove the caps and expose the reverse part of the titanium implant.

All bone were scanned in Skyscan1072 (Skyscan, Aartselaar, Belgium) desktop X-ray CT scanner at 7 µm voxel resolution (50× magnification), X-ray tube current 173 µA and voltage 60 kV with a 0.5 mm aluminum/copper filter. Specimens were mounted vertically on a plastic support and rotated through 180° around the long axis (z-axis) of the sample. Four absorption images were recorded every 0.400° of rotation. These projection radiographs were used in standard cone-beam reconstruction software to generate a series of 1024 8-bit axial slices, each of 1024×1024 pixels, that had Z-dimensional spacing equal to the within slice pixel spacing. The resulting 3-D data sets were hence isotropic with voxel spacing of 7 µm over the entire 1024³ spatial range. All scaffolds were aligned in both horizontal and vertical plane. 3-D reconstruction of the internal pore morphology was carried out using these axial bitmap images and analysed by CTan (Skyscan, Aartselaar, Belgium), where the grey scale threshold was set between 21 and 90. This threshold filtered out the titanium oxide scaffold. Thus the interal pore morphology of the trabecular bone was performed with the scaffold structure as a void. The region of interest was selected as a cylinder (5mm in diameter and 10 mm long) inside the trabecular bone area. The shape of the cylinder was constant for analyses of all samples. The data was later processed with statitical software (SPSS v15 for Windows, US)

### Results

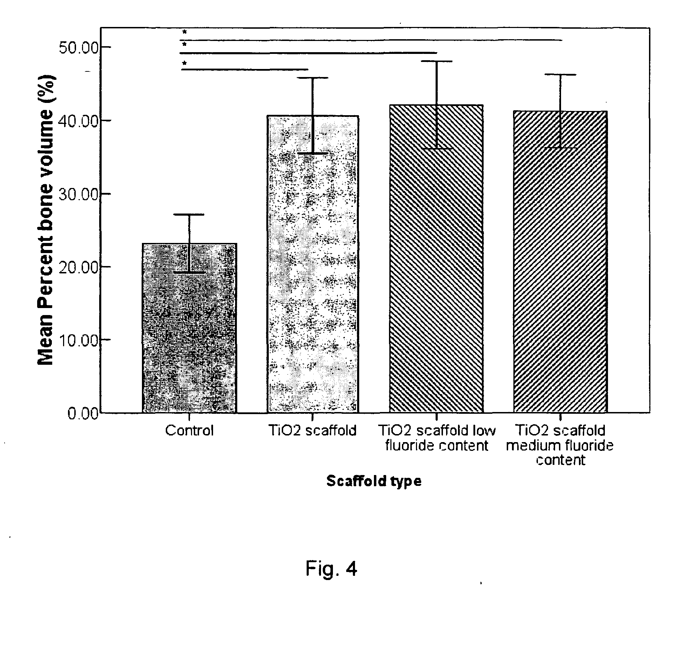

Titanium oxide scaffold, titanium oxide scaffold with low flouride and titanium oxide scaffold with medium flouride content had all highly significantly (p<0.001) increased bone formation inside the scaffold (Fig. 4) when compared to control (sham).

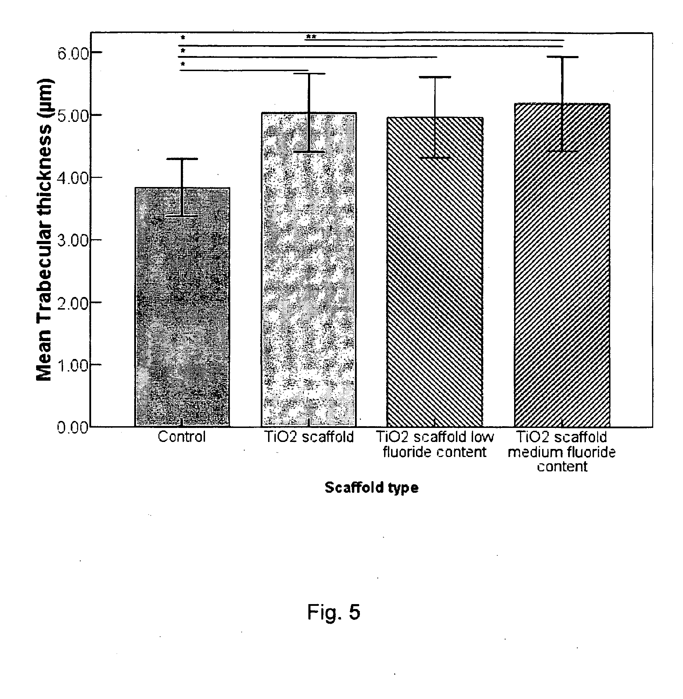

There was a highly significant difference in the quality of the bone inside the scaffold when compared to control (p<0.001), however also a significant difference between the scaffold with medium content of fluoride compared to the pure titanium oxide scaffold (p<0.05) (Fig. 5), which indicated that fluoride stimulated bone maturation and growth.

### EXAMPLE 11: SLURRY RECIPE TITANIUM OXIDE AND ALUMINUM OXIDE COMPOSITE

### Materials and methods:

Titanium oxide and aluminum oxide powder were mixed in dry condition with the ratio of 80 wt.% TiO₂ (Pharma, Sachtleben, Germany) and 20 wt.% Al₂O₃ (No. 713-40 II/973, Fa. Nabaltec, Germany). The mixture was thoroughly homogenised in a mortar and dispersed in a liquid consisting of 24 ml deionised water, 2.0 g 5% methylcellulose solution (Tylopur C 30, Hoechst, Germany) as binder and 0.2 g polycarbonic acid (Dolapix CE 64, Zschimmer & Schwarz, Germany). The slurry was then homogenised with an electrical stirrer for 5 min. In order to achieve optimal particle sizes, the ceramic slurry was dispersed with a high speed dissolver (Dispermat Ca-40, VMA-Getzmann GmbH, Reichshof , Germany) with a custom made titanium rotorblade. The porous structure and sintering was performed as described in examples 8 and 9. In addition to the described weight percentage, also 30 wt.%, 40 wt.% and 50 wt% of Al₂O₃ was tried.

### Results:

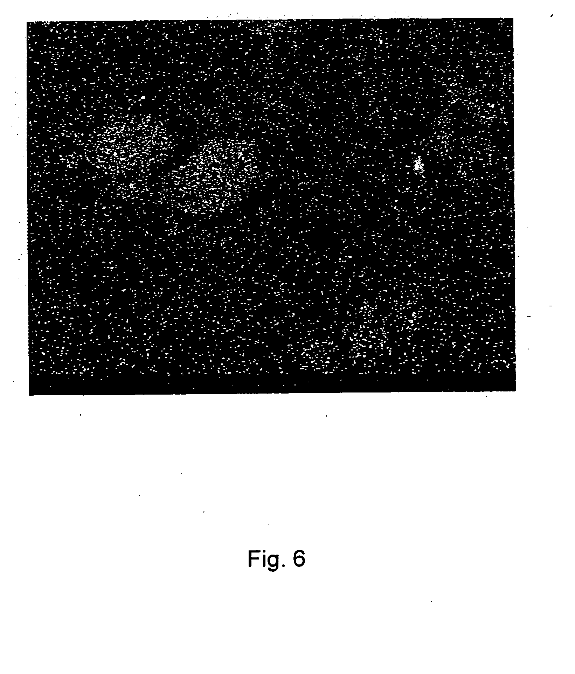

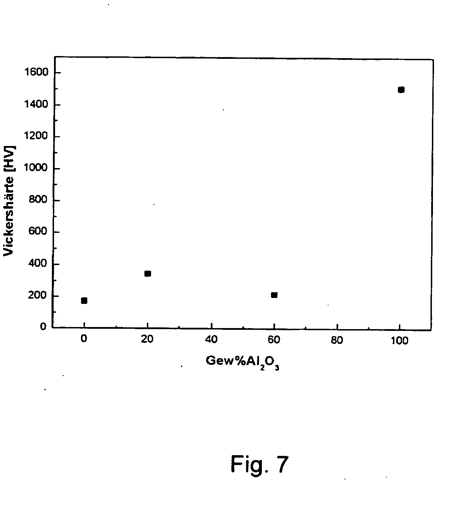

The mixture ratio of 80wt. % TiO₂ and 20 wt.% Al₂O₃ gave the most stable solid object. The different materials are well dispersed, which can be seen in an EDX image (Fig. 6). The hardness of the samples (according to Vickers test) are displayed in Fig. 7.

### EXAMPLE 12: SLURRY RECIPE TITANIUM OXIDE AND ALUMINUM OXIDE COMPOSITE

### Materials and methods:

Titanium oxide and aluminum oxide powder were mixed in dry condition with the ratio of 80wt. % TiO₂ (Pharma, Sachtleben, Germany) and 20 wt. % commercial ZrO₂ powder (3 mol% Y₂O₃, Cerac Inc., WI, USA) was used to prepare a slurry mixture. The powder of 100 g was stirred vigorously in 150 ml distilled water dispersed with a triethyl phosphate (TEP; (C₂H₅)₃PO₄, Aldrich, USA) of 6 g for 24 h. As a binder, poly vinylbutyl (PVB, Aldrich, USA) of 6 g was dissolved in another beaker, which was subsequently added to the slurry and stirred for an additional 24 h. The porous structure and sintering was performed as described in exampleS 8 and 9. In addition to the described weight percentage, also 40 wt.%, 60 wt.% and 80 wt% of ZrO₂ powder was tried.

### Results:

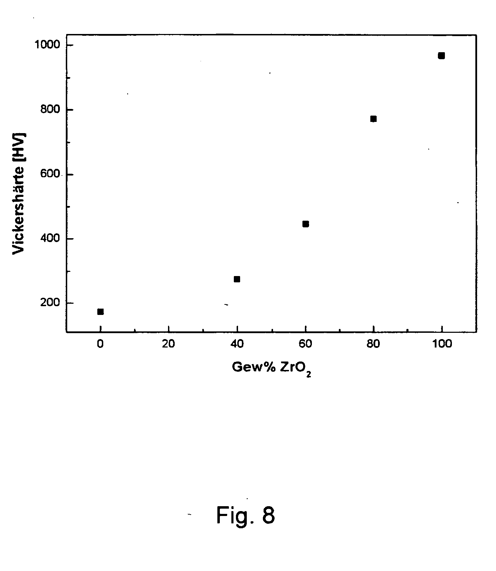

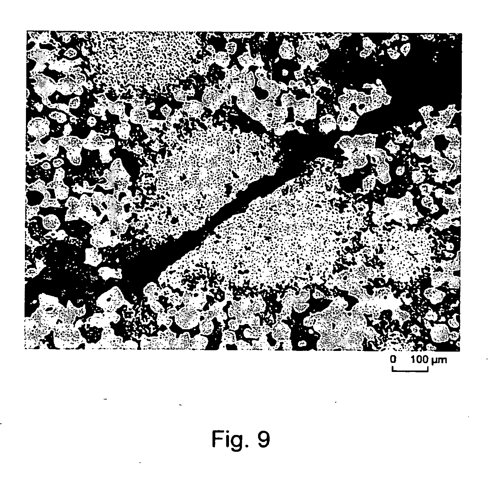

The hardness of the structure increased with increased amount of ZrO₂. The highest hardness was found for samples with 80 wt % ZrO₂. Fig. 8 shows a Vickershardness of mixture of ZrO2 and TiO2 composites. A microscope image is also shown (Fig. 9).

### EXAMPLE 13

The composition of the mixture is a described in example 11 and 12, however the mixing is performed in wet conditions in a ball mill.

### REFERENCES:

1. Iwatsuki S, Honda MJ, Harada H, Ueda M. Cell proliferation in teeth reconstructed from dispersed cells of embryonic tooth germs in a three-dimensional scaffold. European journal of oral sciences 2006 Aug;114(4):310-317.
2. Li WJ, Tuli R, Okafor C, Derfoul A, Danielson KG, Hall DJ, et al. A three-dimensional nanofibrous scaffold for cartilage tissue engineering using human mesenchymal stem cells. Biomaterials 2005 Feb;26(6):599-609.
3. Rodriguez A, Cao YL, Ibarra C, Pap S, Vacanti M, Eavey RD, et al. Characteristics of cartilage engineered from human pediatric auricular cartilage. Plastic and reconstructive surgery 1999 Apr; 103(4): 1111-1119.
4. Shieh SJ, Terada S, Vacanti JP. Tissue engineering auricular reconstruction: in vitro and in vivo studies. Biomaterials 2004 Apr;25(9):1545-1557.
5. Sodian R, Hoerstrup SP, Sperling JS, Martin DP, Daebritz S, Mayer JE, Jr., et al. Evaluation of biodegradable, three-dimensional matrices for tissue engineering of heart valves. Asaio J 2000 Jan-Feb;46(1):107-110.
6. Du C, Cui FZ, Zhu XD, de Groot K. Three-dimensional nano-HAp/collagen matrix loading with osteogenic cells in organ culture. Journal of biomedical materials research 1999 Mar 15;44(4):407-415.
7. Rekow D. Informatics challenges in tissue engineering and biomaterials. Advances in dental research 2003 Dec;17:49-54.
8. Stiehl JB. Trabecular metal in hip reconstructive surgery. Orthopedics 2005 - Jul;28(7):662-670.
9. Nygren P-A, Uhlen M. Scaffolds for engineering novel binding sites in proteins. Current Opinion in Structural Biology 1997 1997/8;7(4):463-469.
10. Nygren H, Tengvall P, Lundstrom I. The initial reactions of TiO2 with blood. Journal of Biomedical Materials Research 1997 1997/3/15;34(4):487-492.
11. Grikscheit TC, Ochoa ER, Ramsanahie A, Alsberg E, Mooney D, Whang EE, et al. Tissue-engineered large intestine resembles native colon with appropriate in vitro physiology and architecture. Annals of surgery 2003 Jul;238(1):35-41.
12. Eckert KL, Mathey M, Mayer J, Homberger FR, Thomann PE, Groscurth P, et al. Preparation and in vivo testing of porous alumina ceramics for cell carrier applications. Biomaterials 2000;21(1):63-69.
13. Haugen H, Will J, Kohler A, Hopfner U, Aigner J, Wintermantel E. Ceramic Ti02-foams: characterisation of a potential scaffold. Journal of the European Ceramic Society 2004;24(4):661-668.
14. Schwartzwalder K, Somers AV, inventors. Method of Making a Porous Shape of Sintered Refractory Ceramic Articles. United States Patent No. 3090094, 1963.
15. Gibson LJ, Ashby MF. Cellular solids - structure and properties. 2. Auflage ed. Cambridge: Cambridge University Press, 1997. p. 205.
16. Luyten J, Cooymans J, De Wilde A, Thijs I. Porous materials, synthesis and characterization. Key Engineering Materials 2002;206-213(2):1937-1940.
17. Peng HX, Fan Z, Evans JRG, Busfield JJC. Microstructure of Ceramic Foams. Journal of the European Ceramic Society 2000 2000/6;20(7):807-813.
18. Powell SJ, Evans J. The structure of ceramic foams prepared from polyurethane ceramic suspensions. Materials and Manufacturing Processes 1995;10(4):757-771.
19. Saggiowoyanski J, Scott CE, Minnear WP. Processing of porous ceramics. American Ceramic Society Bulletin 1992 1992/11;71(11):1674-1682.
20. WO 95/17217
21. WO 94/13334
22. WO 04/008983
23. WO 04/008984
24. Kim H-W, Lee S-Y, Bae C-J, Noh Y-J, Kim H-E, Kim H-M and Ko JS. Porous ZrO2 bone scaffold coated with hydroxyapatite with fluoroapatite intermediate layer. Biomaterials 2003 24: 3277-3284.
25. Jokinen M, Pätsi M, Rahiala H, Peltola T, Ritala M, Rosenholm JB. Influence of sol and surface properties on in vitro bioactivity of sol-gel-derived TiO2 and TiO2-SiO2 films deposited by dip-coating method. Journal of Biomedical Materials Research 1998;42(2):295-302.
26. Nygren H, Tengvall P, Lundstrom I. The initial reactions of Ti02 with blood. Journal of Biomedical Materials Research 1997 1997/3/15;34(4):487-492.
27. Nygren H, Eriksson C, Lausmaa J. Adhesion and activation of platelets and polymorphonuclear granulocyte cells at TiO2 surfaces. The Journal of Laboratory and Clinical Medicine 1997 1997/1;129(1):35-46.
28. Wintermantel E, Cima L, Schloo B, Langer R. Angiopolarity of cell carriers, Directional angiogenesis in resorbable liver cell transplantation devices. In: Steiner R, Weisz B, Langer R, editors. Angiogenesis: Principles-Science-Technology-Medicine. Basel: Birkhaeuser Verlag, 1992.
29. Piskin E. Biomaterials in Different Forms for Tissue Engineering: An Overview. Materials Science Forum 1997;250:1-14.
30. Polonchuk L, Elbel J, L. E, J. B, Wintermantel E, Eppenberger HM. Titanium dioxide ceramics control the differentiated phenotype of cardiac muscle cells in culture. Biomaterials 2000;21 (6):539-550.

## Claims

1. A metal oxide scaffold comprising titanium oxide, said scaffold having a compression strength of about 0.1-150 MPa, wherein said titanium oxide in said metal oxide scaffold constitutes 40-100 wt%, preferably 60-90 wt%, of the metal oxides present in the scaffold, and wherein said titanium oxide comprises less than about 10 ppm of contaminations of secondary and/or tertiary phosphates.

2. A metal oxide scaffold according to claim 1, wherein said compression strength is about 5-15 MPa.

3. A metal oxide scaffold according to any of claims 1-2 having a porosity of about 40-99% preferably 70-90 %.

4. A metal oxide scaffold according to any of the preceding claims having a pore size of about 10-3000 µm, preferably about 20-2000 µm, more preferably about 30-1500 µm and even more preferably about 30-700 µm.

5. A metal oxide scaffold according to any of the preceding claims having a fractal dimension strut of about 2.0-3.0, preferably about 2.2-2.3.

6. A metal oxide scaffold according to any of the preceding claims having an inner strut volume of about 0.001-3.0 µm³, preferably about 0.8-1.2 µm³.

7. A metal oxide scaffold according to any of the preceding claims, wherein the pores are interconnective or partially interconnective.

8. A metal oxide scaffold according to any of the preceding claims, further comprising a least one oxide of Zr, Hf, V, Nb, Ta and/or Al.

9. A metal oxide scaffold according to any of the preceding claims comprising at least one surface which is at least partially covered with fluoride and/or fluorine.

10. A metal oxide scaffold according to claim 9, wherein said fluoride is provided in an aqueous solution comprising HF, NaF and/or CaF₂, in a gas phase and/or as a vapour.

11. A metal oxide scaffold according to any of the preceding claims, wherein the titanium oxide comprises TiO₂.

12. A metal oxide scaffold according to any of the preceding claims, wherein said metal oxide comprises one or more titanium oxides selected from TiO₂, Ti₃O, Ti₂O, Ti₃O₂, TiO, Ti₂O₃, or Ti₃O₅.

13. A metal oxide scaffold according to any of the preceding claims for the regeneration, repair, substitution and/or restoration of tissue, such as bone.

14. A medical implant comprising a metal oxide scaffold according to any of the preceding claims.

15. A method for producing a metal oxide scaffold as defined in any of claims 1-13 comprising the steps of
a) preparing a slurry of metal oxide comprising titanium oxide, said slurry optionally comprising fluoride ions and/or fluorine
b) providing the slurry of step a) to a porous polymer structure
c) allowing the slurry of step b) to solidify
d) removing the porous polymer structure from the solidified metal oxide slurry.

16. A method according to claim 15 wherein step d) is performed by
i) slow sintering of the porous polymer structure with the solidified metal oxide slurry to about 500°C and holding this temperature for at least 30 minutes,
ii) fast sintering to about minimum 1500°C or to about 1750°C at ca 3 K/min and holding this temperature for at least 10 hours, and
iii) fast cooling to room temperature at least 3 K/min.

17. A method according to any of claims 15-16, further comprising the step of treating the metal oxide scaffold with fluoride and/or fluorine.

18. A method according to claim 17, wherein the fluoride and/or fluorine is provided in an aqueous solution comprising HF, NaF and/or CaF₂, in a gas phase and/or as a vapour.

19. A method according to claim 18, wherein the concentration of fluoride and/or fluorine in said solution is 0.001-2.0 wt%, preferably 0.05-1.0 wt%.

20. A metal oxide scaffold according to any of claims 1-13 for use as a medical implant.

21. A metal oxide scaffold according to claim 20 for use for the regeneration of tissue.

22. A metal oxide scaffold according to any of claims 20-21 for use for the regeneration of bone.

23. Use of a metal oxide scaffold according to any of claims 1-13 for the preparation of a medical implant for the regeneration, repair, substitution and/or restoration of tissue and/or bone.

24. A metal oxide scaffold according to any of claims 1-13, which metal oxide scaffold has been granulated, for use as a bone filling material.

25. A slurry for preparing a metal oxide scaffold according to any of claims 1-13 said slurry comprising an aqueous solution of titanium oxide powder, said titanium oxide powder comprising less than about 10 ppm of contaminations of secondary and/or tertiary phosphates.

## Patentansprüche

1. Metalloxidgerüst, das Titanoxid umfasst, wobei das genannte Gerüst eine Druckfestigkeit von etwa 0,1-150 MPa hat, wobei das genannte Titanoxid in dem genannten Metalloxidgerüst 40-100 Gew.%, vorzugsweise 60-90 Gew.%, der in dem Gerüst vorliegenden Metalloxide ausmacht, und wobei das genannte Titanoxid weniger als etwa 10 ppm an Kontaminationen von sekundären und/oder tertiären Phosphaten umfasst.

2. Metalloxidgerüst gemäß Anspruch 1, wobei die genannte Druckfestigkeit etwa 5-15 MPa ist.

3. Metalloxidgerüst gemäß einem der Ansprüche 1-2, das eine Porösität von etwa 40-99%, vorzugsweise 70-90%, hat.

4. Metalloxidgerüst gemäß einem der vorangehenden Ansprüche, das eine Porengröße von etwa 10-3000 µm, vorzugsweise etwa 20-2000 µm, mehr bevorzugt etwa 30-1500 µm und noch mehr bevorzugt etwa 30-700 µm, hat.

5. Metalloxidgerüst gemäß einem der vorangehenden Ansprüche, das eine fraktale Dimensionsverstrebung von etwa 2,0-3,0, vorzugsweise etwa 2,2-2,3, hat.

6. Metalloxidgerüst gemäß einem der vorangehenden Ansprüche, das ein inneres Verstrebungsvolumen von etwa 0,001-3,0 µm³, vorzugsweise etwa 0,8-1,2 µm³, hat.

7. Metalloxidgerüst gemäß einem der vorangehenden Ansprüche, wobei die Poren miteinander verbunden sind oder teilweise miteinander verbunden sind.

8. Metalloxidgerüst gemäß einem der vorangehenden Ansprüche, das weiterhin mindestens ein Oxid von Zr, Hf, V, Nb, Ta und/oder Al umfasst.

9. Metalloxidgerüst gemäß einem der vorangehenden Ansprüche, das mindestens eine Oberfläche, die mindestens teilweise mit Fluorid und/oder Fluor bedeckt ist, umfasst.

10. Metalloxidgerüst gemäß Anspruch 9, wobei das genannte Fluorid in einer wässrigen Lösung, die HF, NaF und/oder CaF₂ umfasst, in einer Gasphase und/oder als ein Dampf bereitgestellt ist.

11. Metalloxidgerüst gemäß einem der vorangehenden Ansprüche, wobei das Titanoxid TiO₂ umfasst.

12. Metalloxidgerüst gemäß einem der vorangehenden Ansprüche, wobei das genannte Metalloxid ein oder mehrere Titanoxid/e, ausgewählt aus TiO₂, Ti₃O, Ti₂O, Ti₃O₂, TiO, Ti₂O₃ oder Ti₃O₅, umfasst.

13. Metalloxidgerüst gemäß einem der vorangehenden Ansprüche für die Regeneration, die Reparatur, den Ersatz und/oder die Wiederherstellung von Gewebe, beispielsweise Knochen.

14. Medizinisches Implantat, das ein Metalloxidgerüst gemäß einem der vorangehenden Ansprüche umfasst.

15. Verfahren zur Herstellung eines Metalloxidgerüsts wie in einem der Ansprüche 1 bis 13 definiert, das die Schritte umfasst von
a) Herstellen einer Aufschlammung des Metalloxids, das Titanoxid umfasst, wobei die Aufschlammung optional Fluoridionen und/oder Fluor umfasst.
b) Bereitstellen der Aufschlämmung aus a) an eine poröse Polymerstruktur
c) Zulassen, dass sich die Aufschlammung aus b) verfestigt
d) Entfernen der porösen Polymerstruktur von der verfestigten Metalloxidaufschlämmung.

16. Verfahren gemäß Anspruch 15, wobei Schritt d) durchgeführt wird durch
i) Langsames Sintern der porösen Polymerstruktur mit der verfestigten Metalloxidaufschlämmung auf etwa 500°C und Halten dieser Temperatur für mindestens 30 Minuten,
ii) Schnelles Sintern auf etwa mindestens 1500°C oder auf etwa 1750°C mit circa 3 K/min und Halten dieser Temperatur für mindestens 10 Stunden, und
iii)Schnelles Abkühlen auf Raumtemperatur mit mindestens 3 K/min.

17. Verfahren gemäß einem der Ansprüche 15-16, das weiterhin den Schritt des Behandelns des Metalloxidgerüsts mit Fluorid und/oder Fluor umfasst.

18. Verfahren gemäß Anspruch 17, wobei das Fluorid und/oder Fluor in einer wässrigen Lösung, die HF, NaF und/oder CaF₂ umfasst, in einer Gasphase und/oder als ein Dampf bereitgestellt wird.

19. Verfahren gemäß Anspruch 18, wobei die Konzentration an Fluorid und/oder Fluor in der genannten Lösung 0,001-2,0 Gew.%, vorzugsweise 0,05-1,0 Gew.%, ist.

20. Metalloxidgerüst gemäß einem der Ansprüche 1 bis 13 zur Verwendung als ein medizinisches Implantat.

21. Metalloxidgerüst gemäß Anspruch 20 zur Verwendung für die Regeneration von Gewebe.

22. Metalloxidgerüst gemäß einem der Ansprüche 20-21 zur Verwendung für die Regeneration von Knochen.

23. Verwendung eines Metalloxidgerüsts gemäß einem der Ansprüche 1 bis 13 für die Herstellung eines medizinischen Implantats für die Regeneration, die Reparatur, den Ersatz und/oder die Wiederherstellung von Gewebe und/oder Knochen.

24. Metalloxidgerüst gemäß einem der Ansprüche 1 bis 13, wobei das Metalloxidgerüst granuliert wurde, zur Verwendung als ein Knochenfüllmaterial.

25. Aufschlammung zur Herstellung eines Metalloxidgerüsts gemäß einem der Ansprüche 1 bis 13, wobei die genannte Aufschlämmung eine wässrige Lösung von Titanoxidpulver umfasst, wobei das genannte Titanoxidpulver weniger als etwa 10 ppm an Kontaminationen von sekundären und/oder tertiären Phosphaten umfasst.

## Revendications

1. Ossature d'oxydes métalliques comprenant de l'oxyde de titane, ladite ossature ayant une résistance à la compression d'environ 0,1 à 150 MPa, dans laquelle ledit oxyde de titane dans ladite ossature en oxydes métalliques représente 40 à 100 % en poids, de préférence 60 à 90 % en poids, des oxydes métalliques présents dans l'ossature, et dans laquelle ledit oxyde de titane comprend moins d'environ 10 ppm de contaminants consistant en phosphates secondaires et/ou tertiaires.

2. Ossature d'oxydes métalliques suivant la revendication 1, dans laquelle ladite résistance à la compression est d'environ 5 à 15 MPa.

3. Ossature en oxydes métalliques suivant l'une quelconque des revendications 1 et 2, ayant une porosité d'environ 40 à 99 %, de préférence de 70 à 90 %.

4. Ossature d'oxydes métalliques suivant l'une quelconque des revendications précédentes, ayant un diamètre de pore d'environ 10 à 3000 µm, avantageusement d'environ 20 à 2000 µm, plus avantageusement d'environ 30 à 1500 µm et encore plus avantageusement d'environ 30 à 700 µm.

5. Ossature d'oxydes métalliques suivant l'une quelconque des revendications précédentes, ayant une entretoise de dimension fractale d'environ 2,0 à 3,0, de préférence d'environ 2,2 à 2,3.

6. Ossature d'oxydes métalliques suivant l'une quelconque des revendications précédentes, ayant un volume d'entretoise interne d'environ 0,001 à 3,0 µm³, de préférence d'environ 0,8 à 1,2 µm³.

7. Ossature d'oxydes métalliques suivant l'une quelconque des revendications précédentes, dans laquelle les pores présentent une interconnexion ou interconnexion partielle.

8. Ossature d'oxydes métalliques suivant l'une quelconque des revendications précédentes, comprenant en outre au moins un oxyde de Zr, Hf, V, Nb, Ta et/ou Al.

9. Ossature d'oxydes métalliques suivant l'une quelconque des revendications précédentes, comprenant au moins une surface qui est couverte au moins partiellement avec un fluorure et/ou du fluor.

10. Ossature d'oxydes métalliques suivant la revendication 9, dans laquelle ledit fluorure est fourni dans une solution aqueuse comprenant Hf, NaF et/ou CaF₂, dans une phase gazeuse et/ou sous forme de vapeur.

11. Ossature d'oxydes métalliques suivant l'une quelconque des revendications précédentes, dans laquelle l'oxyde de titane comprend TiO₂.

12. Ossature d'oxydes métalliques suivant l'une quelconque des revendications précédentes, dans laquelle ledit oxyde métallique comprend un ou plusieurs oxydes de titane choisis entre TiO₂, Ti₃O, Ti₂O, Ti₃O₂, TiO, Ti₂O₃ et Ti₃O₅.

13. Ossature d'oxydes métalliques suivant l'une quelconque des revendications précédentes, pour la régénération, la réparation, la substitution et/ou la reconstitution d'un tissu, tel que le tissu osseux.

14. Implant médical comprenant une ossature d'oxydes métalliques suivant l'une quelconque des revendications précédentes.

15. Procédé pour produire une ossature d'oxydes métalliques telle que définie dans l'une quelconque des revendications 1 à 13, comprenant les étapes suivantes :
a) préparer une suspension d'oxyde métallique comprenant de l'oxyde de titane, ladite suspension comprenant éventuellement des ions fluorure et/ou du fluor
b) fournir la suspension de l'étape a) à une structure polymère poreuse
c) laisser la suspension de l'étape b) se solidifier
d) éliminer la structure polymère poreuse de la suspension d'oxyde métallique solidifiée.

16. Procédé suivant la revendication 15, dans lequel l'étape d) est mise en oeuvre par
i) frittage lent de la structure polymère poreuse avec la suspension d'oxyde métallique solidifiée à environ 500°C et maintien de cette température pendant au moins 30 minutes,
ii) frittage rapide à une température minimale d'environ 1500°C ou à environ 1750°C à environ 3 K/min et maintien de cette température pendant au moins 10 heures, et
iii) refroidissement rapide à température ambiante à au moins 3 K/min.

17. Procédé suivant l'une quelconque des revendications 15 et 16, comprenant en outre l'étape de traitement de l'ossature d'oxydes métalliques avec un fluorure et/ou du fluor.

18. Procédé suivant la revendication 17, dans lequel le fluorure et/ou le fluor est fourni dans une solution aqueuse comprenant HF, NaF et/ou CaF₂, dans une phase gazeuse et/ou sous forme de vapeur.

19. Procédé suivant la revendication 18, dans lequel la concentration en fluorure et/ou du fluor dans ladite solution va de 0,001 à 2,0 % en poids, de préférence de 0,05 à 1,0 % en poids.

20. Ossature d'oxydes métalliques suivant l'une quelconque des revendications 1 à 13, pour une utilisation commue implant médical.

21. Ossature d'oxydes métalliques suivant la revendication 20, pour une utilisation pour la régénération d'un tissu.

22. Ossature d'oxydes métalliques suivant l'une quelconque des revendications 20 et 21, pour une utilisation pour la régénération du tissu osseux.

23. Utilisation d'une ossature d'oxydes métalliques suivant l'une quelconque des revendications 1 à 13, pour la préparation d'un implant médical pour la régénération, la réparation, la substitution et/ou la reconstitution d'un tissu et/ou du tissu osseux.

24. Ossature d'oxydes métalliques suivant l'une quelconque des revendications 1 à 13, ossature d'oxydes métalliques qui a été granulée, pour une utilisation commue matériau de comblement du tissu osseux.

25. Suspension pour préparer une ossature d'oxydes métalliques suivant l'une quelconque des revendications 1 à 13, ladite suspension comprenant une solution aqueuse de poudre d'oxyde de titane, ladite poudre d'oxyde de titane comprenant moins d'environ 10 ppm de contaminants consistant en phosphates secondaires et/ou tertiaires.
